(19) Europäisches Patentamt
European Patent Office
Office européen des brevets

(11) Veröffentlichungsnummer : **0 117 274**
**B1**

(12) # EUROPÄISCHE PATENTSCHRIFT

(45) Veröffentlichungstag der Patentschrift : **12.11.86**

(51) Int. Cl.⁴ : **A 61 K 31/635**, A 61 K 31/505, A 61 K 9/22

(21) Anmeldenummer : **83107161.8**

(22) Anmeldetag : **21.07.83**

(54) **Kaliumneutrales Saluretikum mit antihypertensiver Wirkung.**

(30) Priorität : **21.02.83 DE 3305935**

(43) Veröffentlichungstag der Anmeldung : **05.09.84 Patentblatt 84/36**

(45) Bekanntmachung des Hinweises auf die Patenterteilung : **12.11.86 Patentblatt 86/46**

(84) Benannte Vertragsstaaten : **AT BE CH DE FR GB IT LI LU NL SE**

(56) Entgegenhaltungen :
**AT-B- 344 914**
**AT-B- 365 452**
**DE-B- 2 615 694**
**GB-A- 2 056 278**
**US-A- 3 081 230**
**US-A- 3 532 792**
**THERAPIEWOCHE, 30. Jahrgang, 1980/IV VERLAG G. BRAUN "Untersuchungen zum verbesserten therapeutischen Einsatz kaliumsparender Diuretika" Seiten 6831-6847**
**PHARMAZIE, Jahrgang 34, Heft 8, 1979, Berlin VEB VERLAG VOLK UND GESUNDHEIT "Anwendung und Entwicklung von Antihypertonica" Seiten 481-486**

(73) Patentinhaber : **MEDICE Chem.-Pharm. Fabrik Pütter GmbH & Co. KG**
**Postfach 415**
**D-5860 Iserlohn/Westf. (DE)**

(72) Erfinder : **Pütter, Sigurd,Dr.**
**Kuhloweg 46**
**D-5860 Iserlohn/Westf. (DE)**

(74) Vertreter : **Brauns, Hans-Adolf, Dr. rer. nat. et al**
**Hoffmann, Eitle & Partner, Patentanwälte Arabellastrasse 4**
**D-8000 Munich 81 (DE)**

**EP 0 117 274 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein kaliumneutrales Saluretikum mit antihypertensiver Wirkung das eine Kombination von zwei bekannten Diuretika, nämlich Furosemid und Triamteren enthält.

Furosemid hat die Formel

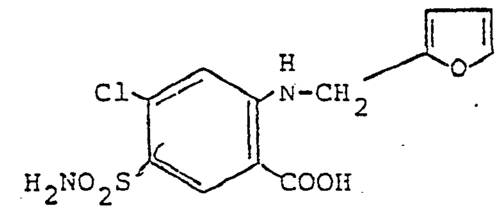

Triamteren hat die Formel

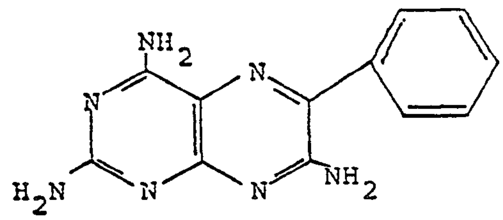

Furosemid und Entwicklungen wie Bumetanid sowie aus Substanzklassen wie Mutzolimin sind vorteilhaft für die Oedem- und Hochdruckbehandlung geeignet. Diesen letzteren Substanzen sind vor allem der schnelle Wirkungseintritt, eine kurze Wirkungsdauer mit Übergang in ein sogenanntes Rebound-Phänomen und die sehr starke Gesamtwirkung eigen. Ursache hierfür ist die kurze Halbwertszeit von ca. 1 h gegenüber 4 h bei den Benzothiadiazindioxid-Derivaten (Kurzbezeichnung Thiazide). Folge der sehr starken und abrupten Wirkung sind Kreislaufbeschwerden, die sogar einen Kollaps auslösen können. Weitere unerwünschte Arzneimittelwirkungen sind u. a. Anstieg der Harnsäure, diabetogene Wirkung, Beeinflussung des Fettstoffwechsels, starke Kaliumverluste und massive Stimulierung des Plasmarenin-Angiotensin-Systems (Tabelle 1).

Dagegen ist der Wirkungseintritt bei Triamteren langsamer und die Wirkungsdauer mittellang und die diuretische Wirkung nur schwach. Dafür werden die Kalium- und Magnesiumausscheidung unter die Norm gesenkt und der Kohlenhydrat-Stoffwechsel nicht beeinflußt (Tabelle 1).

Die Suche nach einem sogenannten idealen Diuretikum, das einersetis stark natriuretisch wirkt und andererseits einen Kaliumverlust verhindert, war bisher vergebens und wird vermutlich auch aufgrund der funktionellen Heterogenität des Nephrons und der intrarenalen Wirkungsmechanismen aussichtslos bleiben. In dieser Situation bieten sich nur Kombinationen von Diuretika an, die sich aufgrund ihrer renalen Wirkungsorte und damit ihrer Wirkweise unterscheiden und möglichst ergänzen.

Aus der DE-AS 26 15 694 ist die Kombination von Triamteren mit Cyclothiazid bekannt. Bei Reduktion der Einzeldosierung wurden stärkere Gesamtwirkungen und auch weniger unerwünschte Arzneimittelnebenwirkungen, vor allem bei längerem Gebrauch, beobachtet. Voraussetzung für eine ideale Kombination ist jedoch, daß beide Partner in der pharmakokinetischen Halbwertszeit und der Pharmakodynamik übereinstimmen.

Aus Therapiewoche 30, 1980, Seiten 6831 bis 6847 ist bekannt, daß durch gleichzeitige i. v.-Gabe von Furosemid und Hydroxitriamteren-Schwefelsäureester, der eine etwa 10-fach höhere Plasmakonzentration erreicht als Triamteren, der durch Furosemid bedingte Kaliumverlust verhindert werden kann (vgl. auch AT-A 365 452).

Aufgabe der Erfindung ist es ein kaliumneutrales Saluretikum mit antihypertensiver Wirkung zur Verfügung zu stellen das die folgenden Eigenschaften aufweist :

1. Initial bald einsetzende Wirkung.
2. Keine abrupte und kurze Wirkung, d. h. kein Rebound.
3. Möglichst protrahierte Wirkung über 10 Stunden.
4. Gute und gleichzeitig schonende Ausschwemmung ohne Kreislaufbelastung und Thrombosegefahr.
5. Wirkung auch bei eingeschränkter Nierenfunktion : Clearance : ≤ 30 ml/min ; ein Bereich, bei dem Thiazide nicht mehr wirksam sind.
6. Kaliumneutral ; Magnesium- und Kalziumionen sparend.
7. Möglichst ohne Einfluß auf Kohlenhydrat- und Fett-Stoffwechsel sowie Harnsäureausscheidung.

Voraussetzung für die Lösung dieser Aufgabe ist es, daß man ein sogenanntes Schleifendiuretikum mit einem Kaliumsparer kombiniert, und daß folgende Bedingungen erfüllt sind :

1. Angleichung der pharmakokinetischen Halbwertszeiten durch verzögerte Freisetzung.

2. Angleichung der pharmakodynamischen Zeiten.

Für Furosemid trifft dies jedoch aufgrund der kurzen Halbwertszeit von ca. 1 h nicht zu, wie aus Untersuchungen von H. GRAUL und H. KAFFARNIK hervorgehen (Tabelle 2). Eine Kombination aus einer konstanten Dosis Furosemid mit steigenden Dosen an Triamteren (25 bis 75 mg) zeigt keinen wesentlichen Anstieg der Harn- und Natriumausscheidung im Vergleich zu Furosemid ; die Ausscheidung an Kalium-, Chlor-, Magnesium- und Kalziumionen wird jedoch vermindert.

Ein weiterer Fortschritt läßt sich nur dadurch erreichen, daß Furosemid in einer besonderen galenischen Zubereitung appliziert wird, aus der es einerseits gut und rasch bioverfügbar ist und darüberhinaus eine längere pharmakokinetische und pharmakodynamische Halbwertszeit aufweist (Tabelle 1).

Überraschenderweise wurde nun festgestellt, daß die vorgenannte Aufgabe gelöst werden kann durch die Kombination von Furosemid in Retardform mit Triamteren. Die Erfindung betrifft somit ein kaliumneutrales Saluretikum mit antihypertensiver Wirkung auf Basis von Furosemid und Triamteren bei dem Furosemid in Retardform vorliegt mit einer Wirkstoffreigabe von 5 bis 10 % nach 1 Stunde bis > 85 % ach 8 h und der Wirkstoff Furosemid in einer Menge von 10 bis 50 mg und der Wirkstoff Triamteren in einer Menge von 20 bis 60 mg vorliegt. Die erfindungsgemäße Kombination ergibt eine synergistische Wirkung.

Im Gegensatz zu Furosemid besitzt Furosemid in Retardform eine eindeutig längere und für die Kombination mit Triamteren günstigere Plasmahalbwertszeit. Wie die Untersuchungen zeigen, wird der maximale Plasmaspiegel erst nach 3 bis 4 h erreicht. Zwischen der 6. und 8. Stunde sind noch ausreichende Plasmakonzentrationen an Furosemid in Retardform nachweisbar (Fig. 1a). Bei gleicher Dosierung ist die Kombination Furosemid in Retardform mit Triamteren deutlich wirksamer als Furosemid bzw. Furosemid in Retardform (Tabelle 3).

Auch die Wirkungsdynamik ist besser. Während die Wirkung von Furosemid relativ rasch einsetzte und nur ca. 9 h anhält und anschließend in ein Rebound-Phänomen überging, setzte die Wirkung der Kombination Furosemid in Retardform + Triamteren auch bald ein, das Wirkungsmaximum wird etwas später und nicht so abrupt erreicht, die Wirkungsdauer ist eindeutig länger und klingt erst nach 10 bis 12 h ab (Fig. 2). Diese Wirkungsdynamik ist für den Patiernten kreislaufschonender, weniger belastend und auch komplikationsärmer, insbesondere wenn man berücksichtigt, daß es infolge der massiven Volumensverluste zu einer Hämokonzentration, Erhöhung der Blutviskosität und Thrombosegefährdung kommt.

Völlig unerwartet ist die enorme Verbesserung des Natrium-Kalium-Quotienten, d. h. das Verhältnis von ausgeschiedenem Natrium zu Kalium, ein wichtiger Parameter zur Beurteilung der Qualität eines Diuretikums. Unter Furosemid lag der $NA^+/K^+$ im Untersuchungszeitraum über 12 h im Mittel zwischen 4,1 und 8,2 und zum Zeitpunkt der maximalen Diurese bei 8,2 (Tabelle 3).

Unter Furosemid in Retardform werden Werte zwischen 2,4 und 6,8 erreicht und zum Zeitpunkt der maximalen Diurese Werte zwischen 4,1 und 5,3 (Tabelle 3).

Die Kombination Furosemid in Retardform + Triamteren erzielt Werte zwischen 6,2 und 14,5. Zum Zeitpunkt der maximalen Wirkung liegt der Wert um 14,5 (Tabelle 3).

Überraschenderweise wird zusätzlich durch die Kombination Furosemid in Retardform und Triamteren die Ausscheidung von Magnesium und Kalzium gegenüber Furosemid deutlich gesenkt ; ein Effekt, der besonders bei einer Langzeitbehandlung von Bedeutung ist, da ein Mangel an diesen Elektrolyten u. a. zu Muskelkrämpfen (z. B. Wadenkrämpfe, Tetanie) führen kann.

Bekanntlich stimulieren Schleifendiuretika wie Furosemid infolge der starken Salzverluste und Volumenverschiebungen sehr stark das Plasmarenin-Aldosteron-System. Diese unerwünschte Reaktion konnte überraschenderweise durch die Kombination Furosemid in Retardform + Triamteren erheblich abgeschwächt werden (Fig. 3 und 4).

Bei der Thiazid/Triamteren-Kombination sind gewisse Voraussetzungen erfüllt ; so stimmen die Plasma-Halbwertszeiten weitgehend überein. Durch Reduktion der Einzeldosen wird in der Kombination eine mittelstarke Gesamtwirkung bei einer Kaliumneutralität erreicht. Nicht verbessert werden konnte jedoch die Beeinflussung des Harnsäure- und Fettstoffwechsels. Nicht befriedigend ist vor allem der Einsatz der Thiazid/Triamteren-Kombination bei Patienten mit eingeschränkter Nierenfunktion und älteren Personen, bei denen mit zunehmendem Alter die Nierenleistung nachläßt. Hauptgrund hierfür ist die Tatsache, daß die Thiazide ab einer GFR (Glomerulumfiltration) ≤ 30 ml/min nicht mehr wirken. Aus diesem Grund profitieren gerade die älteren Personen mit Wassereinlagerungen. beispielsweise infolge einer chronischen Herzinsuffizienz oder Patienten mit einem Hochdruck weniger von dieser Kombination. Auch Beta-Rezeptorenblocker sind bei Patienten mit Bradykardie, Herzinsuffizienz und Bronchospasmus problematisch bzw. kontraindiziert.

Die Vorteile der erfindungsgemäßen Kombination aus Furosemid in Retardform und Triamteren sind die synergistischen Wirkungen für die Natrium- und Wasserausscheidung und die antagonistischen Effekte im positiven Sinne, wie u. a. verminderte Magnesium- und Kalziumausscheidung. Gegenüber der bekannten Kombination aus Thiazid und Triamteren ergeben sich folgende Vorteile :

1. Niedrige Einzeldosierung der beiden Substanzen.

2. Infolge der Addition gewünschter diuretischer und natriuretischer Effekte der Einzelkomponenten

besteht ein Synergismus, so daß eine Tagesdosis der Kombination Furosemid in Retardform + Triamteren ausreicht im Gegensatz zu 2 × 1 Tablette Thiazid/Triamteren.

3. Mit diesem Synergismus und dem ausreichend langen Wirkungsprofil wird bei der Einnahme von 1 Dosis eine bessere Compliance erreicht.

4. Gleichzeitig werden die unerwünschten Arzneimittelnebenwirkungen deutlich reduziert, so besteht mit der erfindungsgemäßen Kombination u. a. eine klinisch sehr wertvolle und positiv zu beurteilende Kaliumneutralität und verminderte Magnesium- und Kalziumausscheidung. Der erzielte Natrium-Kalium-Quotient ist eindeutig besser als die in der Literatur mitgeteilten Ergebnisse mit Furosemid bzw. Thiaziden + Triamteren.

5. Im Gegensatz zu der Kombination Thiazid + Triamteren ist die Kombination von Furosemid in Retardform + Triamteren auch bei Patienten mit eingeschränkter GFR noch einsetzbar. Hierbei handelt es sich vor allem um ältere Hypertoniker und ältere Patienten mit Wassereinlagerungen jeglicher Genese.

Die Vorteile der Erfindung gemäß der Kombination Furosemid in Retardform + Triamteren gegenüber der Kombination Furosemid + Triamteren sind die folgenden :

1. Anpassung der pharmakokinetischen Halbwertszeit durch verzögerte Freisetzung von Furosemid.

2. Additive Wirkung durch angepaßte pharmakodynamische Halbwertszeiten ; Furosemid + Triamteren (Tabelle 2), Furosemid in Retardform + Triamteren (Tabelle 3).

3. Bessere Natrium-Neutralität mit einem Natrium-Kalium-Quotienten von 10 bis 14 für Furosemid in Retardform + Triamteren ; für Furosemid + Triamteren nur 4,1.

4. Nur bei Verwendung von Furosemid in Retardform in Kombination mit Triamteren wird die additive Wirkung eine synergistische.

Die beiden Wirkstoffe werden vorzugsweise in Gelatinekapseln kombiniert und liegen in diesen Kapseln vorzugsweise als Granulat oder Pellets vor.

Die Retardierung des Furosemids erfolgt in an sich bekannter Weise, z. B. indem der Wirkstoff mit geeigneten Polymeren gecoatet oder zu einer Einbettung verarbeitet wird. Auch die Verwendung eines Ionenaustauschers oder die Mikroverkapselung des Furosemids bzw. die Herstellung von Hydrokolloid-Matrix-Tabletten — bzw. Pellets — ist möglich. Wesentlich ist, daß eine kontrollierte Wirkstoffreigabe im Bereich von 5 bis 10 % nach 1 h bis > 85 % nach 8 h vorliegt.

Furosemid-Zubereitungen mit einer Freigabeverzögerung des Wirkstoffs sind z. B. in der AT-A 344 914 beschrieben.

Obwohl die Kombination der Wirkstoffe vornehmlich in einer Hartgelatine-Kapsel appliziert wird, sind auch andere orale Verabreichungsformen möglich. So kann man in an sich bekannter Weise Gerüst-Formkörper (Tabletten oder Dragees), aber auch trinkbare Suspensionen sowie Suppositorien, die nach üblichen bekannten Verfahren hergestellt werden können, anwenden. Daneben liegen neben den Wirkstoffen übliche Exzipientien wie beispielsweise Talkum, koloidales Siliziumdioxid, Gummi arabicum, Lactose, Stärke, Zellulosepulver und Magnesiumstearat. Schließlich ist es auch möglich weitere Wirkstoffe in der erfindungsgemäßen Kombination vorzusehen.

## Beispiel

Eine Gelatinekapsel 2 wird mit 30 mg Furosemid in Retardform und 50 mg Triamteren gefüllt. Das Furosemid liegt in Form von Pellets vor, die ausser den 30 mg Furosemid enthalten :

| | |
|---|---|
| Saccharose | 30,2 mg |
| Maisstärke | 5,3 mg |
| Stearinsäure | 0,04 mg |
| Natrium-Carboxymethylcellulose | 0,6 mg |
| Lösliches Polyvinylpyrrolidon | 1,5 mg |
| Schellack | 1,27 mg |
| Talkum | 0,9 mg |

Triamteren liegt in Form von Pellets vor die außer 50 mg des Wirkstoffes enthalten :

| | |
|---|---|
| Saccharose | 35,4 mg |
| Maisstärke | 6,3 mg |
| Stearinsäure | 0,04 mg |
| Natrium-Carboxymethylcellulose | 4,2 mg |
| Lactose | 20,8 mg |
| lösliches Polyvinylpyrrolidon | 8,3 mg |

(Siehe Tabellen Seite 5 ff.)

Pharmakologische Untersuchungen

Tabelle 1

Vergleich verschiedener Diuretika bzw. Diuretika-Kombinationen

0 = keine Beeinflussung
— = Hemmung
↑ ↑ ↑ = Anstieg : sehr stark, stark, deutlich, schwach
↓ = Reduktion
? = Widersprüchlich, nicht belegt

| | Thiazid-Derivate | Furosemid | Triamteren | Thiazid-Triamteren | Furosemid retard Triamteren |
|---|---|---|---|---|---|
| Wirkungseintritt | langsam | schnell | langsam | langsam | bald |
| Wirkungsdauer | mittellang | kurz | mittellang | mittellang | mittellang |
| Gesamtwirkung | gut | sehr stark | schwach | mittelstark | stark |
| Urinvolumen | ↑ | ↑↑↑ | (↑) | ↑(↑) | ↑↑ |
| Natrium | ↑ | ↑↑↑ | (↑) | ↑(↑) | ↑↑ |
| Chlorid | ↑ | ↑↑↑ | (↑) | ↑(↑) | ↑↑ |
| Kalium | ↑ | ↑↑ | — | ↑ — ~ o | ↑ — ~ o |
| Calzium | ↑ ↓ —? | ↑↑ | — | ↓ — = (↑) | ↑↑ — =(↑) |
| Magnesium | ↑ | ↑ | — | ↑ — ~ o | ↑ — ~ o |
| Glucose | ↑ | ↑ | o | o | ? o |
| Harnsäure | ↑ | ↑ | o | ↑ | (↑) |
| Cholesterin | ↑ | ↑ | | ↑ | |
| VLDL / LDL | ↑/↑ | ↑/o | | | |
| HDL | o | o | | | |
| Triglyzeride | ↑ | ↑ | | | |
| GFR | ↓ | o | | ↓ | o |
| Aldosteron | (↑) ? | ↑↑ | | | (↑) |
| Plasmarenin | (↑) ? | ↑↑ | | | (↑) |
| Plasma t 1/2 | 4 Std. | ca. 1 Std. | 3 Std. | 4 Std.<br>3 Std. | 2-3 Std.<br>3 Std. |
| $Na^+/K^+$ | | bis ca. 8 | bis ≥ 20 | bis 10-14 | bis 14,5 |

| | | |
|---|---|---|
| **Wirkung** (synergistisch und antagonistisch im positiven Sinne) | + | + |
| **Vorteil** | | |
| 1. Einzelsubstanz-Einsparung | + | ++ |
| 2. Tagesdosis | 2-1 Tabl. | 1 Kaps. |
| 3. Compliance | + | ++ |
| 4. Reduktion der u.A.W. | + | + |
| 5. eingeschränkte Nierenfunktion | — | + |
| 6. $Na^+/K^+$ | + | ++ |

Tabelle 2

Harn- und Elektrolytausscheidung, sowie Natrium-Quotient bei 7 gesunden männlichen Probanden, die im Abstand von 4 Tagen entweder Furosemid alleine bzw. zusätzlich die verschiedenen Dosen von Triamteren (25-75 mg) erhielten. Als Kontroll-Kollektiv dienten 4 gesunde Probanden. Angegeben sind Mittelwert (X) und Standardabweichung. (Unveröffentliche Befunde : Prof. Dr. Dr. E. H. Graul und Prof. Dr. H. Kaffarnik)

| Zeit | 2 Std. | | | | | 4 Std. | | | | | 8 Std. | | | | |
|---|---|---|---|---|---|---|---|---|---|---|---|---|---|---|---|
| Parameter | Kontrolle | Fu 25 mg | Fu 25mg Tri 25mg | Fu 25mg Tri 50mg | Fu 25mg Tri 75mg | Kontrolle | Fu 25 mg | Fu 25mg Tri 25mg | Fu 25mg Tri 50mg | Fu 25mg Tri 75mg | Kontrolle | Fu 25 mg | Fu 25mg Tri 25mg | Fu 25mg Tri 50mg | Fu 25mg Tri 75mg |
| $U_V$ ml | 229 ± 78 | 927 ± 180 | 539 ± 133 | 524 ± 73 | 546 ± 131 | 452 ± 109 | 1389 ± 198 | 1043 ± 125 | 920 ± 153 | 975 ± 91 | 685 ± 136 | 1650 ± 205 | 1398 ± 162 | 1201 ± 153 | 1403 ± 151 |
| $U_{Na}$ m mol/l | 23 ± 3,6 | 108 ± 19 | 66 ± 10 | 66 ± 3,8 | 78 ± 16 | 49 ± 4,2 | 163 ± 21 | 137 ± 16 | 119 ± 12 | 147 ± 12 | 86 ± 9,7 | 192 ± 18 | 180 ± 19 | 159 ± 16 | 193 ± 21 |
| $U_K$ m mol/l | 19,6 ± 2,0 | 31,5 ± 5,9 | 20,6 ± 5,0 | 22,3 ± 7,3 | 19,5 ± 4,0 | 35,7 ± 1,3 | 51,3 ± 4,8 | 38,1 ± 7,4 | 36,0 ± 9,0 | 33,6 ± 5,3 | 55,5 ± 3,2 | 77,4 ± 5,5 | 62,2 ± 8,5 | 51,3 ± 8,0 | 51,2 ± 8,8 |
| $U_{Na^+/K^+}$ | 1,17 | 3,43 | 3,20 | 2,96 | 4,11 | 1,37 | 3,18 | 3,40 | 3,31 | 4,38 | 1,55 | 2,48 | 2,89 | 3,10 | 3,77 |
| $U_{Cl}$ m mol/l | 31 ± 1 | 131 ± 21 | 70 ± 13 | 64 ± 7 | 80 ± 18 | 60 ± 6 | 206 ± 24 | 147 ± 21 | 115 ± 14 | 151 ± 20 | 93 ± 12 | 242 ± 23 | 192 ± 22 | 154 ± 17 | 199 ± 27 |
| Mg m mol/l | 0,35 ± 0,09 | 1,22 ± 0,36 | 0,54 ± 0,08 | 0,67 ± 0,21 | 0,63 ± 0,18 | 0,65 ± 0,14 | 1,74 ± 0,50 | 1,01 ± 0,13 | 1,06 ± 0,33 | 1,10 ± 0,22 | 1,03 ± 0,20 | 2,08 ± 0,47 | 1,53 ± 0,18 | 1,20 ± 0,26 | 1,72 ± 0,27 |
| Ca m mol/l | 0,41 ± 0,09 | 1,55 ± 0,22 | 1,05 ± 0,21 | 1,05 ± 0,11 | 1,09 ± 0,18 | 0,88 ± 0,18 | 2,42 ± 0,34 | 1,93 ± 0,35 | 1,77 ± 0,25 | 2,00 ± 0,24 | 1,39 ± 0,27 | 2,89 ± 0,38 | 2,56 ± 0,39 | 2,28 ± 0,24 | 2,97 ± 0,34 |

Tabelle 3

Urin- und Elektrolytausscheidung sowie Änderungen im Natrium-Kalium-Quotient, sowie Urin-pH bei je 6 gesunden Probanden nach Einnahme von Furosemid (30 mg), Furosemid retard (30 mg), sowie der Kombination Furosemid retard (30 mg) + Triamteren (50 mg). Angegeben sind Mittelwerte (X) und SM der jeweiligen Absolutwerte bzw. Differenzen (Δ) zum Vortag, sowie die Signifikanzen. Die an Kopf der Zahl angegebenen Kreuze bedeuten Unterschiede zu Furosemid und die an der Basis Unterschiede zu Furosemid retard.

x = p ≤ 0,05, xx ≤ p = 0,01

| Parameter | Substanz | 1,5 Std. | 3 Std. | 4,5 Std. | 6 Std. | 7,5 Std. | 9 Std. | 10,5 Std. | 12 Std. |
|---|---|---|---|---|---|---|---|---|---|
| $U_{pH}$ | Fu. | 5,4 ± 0,17 | 5,0 ± 0,22 | 5,4 ± 0,26 | 6,2 ± 0,23 | 6,0 ± 0,06 | 6,7 ± 0,12 | 6,4 ± 0,12 | 6,9 ± 0,14 |
| | Fu.ret. | 5,4 ± 0,06 | 6,1 ± 0,57 | 5,2 ± 0,13 | 5,9 ± 0,30 | 6,4 ± 0,22 | 6,6 ± 0,20 | 6,3 ± 0,21 | 6,9 ± 0,16 |
| | Fu.ret./Tri | 6,6 ± 0,21 | 7,5 ± 0,29 | 6,0 ± 0,30 | 7,0 ± 0,22 | 7,2 ± 0,16 | 7,1 ± 0,15 | 6,3 ± 0,26 | 6,0 ± 0,18 |
| Δ $U_V$ ml | Fu. | 62,0 ±29,1 | 302,5±103,6 | 167,5 ±109,7 | 92,0±46,3 | 40,5±64,7 | 15,0±33,8 | -87,0±29,0 | -71,3±43,9 |
| | Fu.ret. | 18,5 ±5,9 | 2,5 ±15,2 | 78,8 ±20,3 | 106,0±14,2 | 131,0±23,4 | 105,0±39,6 | 40,7±25,5 | 61,5±41,5 |
| | Fu.ret./Tri | 64,0±10,0 | 243,2±59,9 | 240,7 ±23,7 | 180,2±27,2 | 138,5±45,8 | 108,7±35,2 | 5,2±37,6 | -30,5±19,5 |
| Δ $U_{Na}$ m mol | Fu. | 7,8±3,0 | 28,4 ±6,4 | 20,7±8,6 | 9,3±4,1 | 6,2 ±2,0 | 3,9 ±1,9 | -6,8±2,0 | -3,3 ±1,8 |
| | Fu.ret. | 0,3±1,4 | 0,3 ±1,8 | 10,2±4,0 | 7,3±1,4 | 2,4 ±1,0 | 5,5 ±1,9 | -1,8±2,0 | -0,02±1,8 |
| | Fu.ret./Tri | 7,9±1,2 | 14,2 ±2,7 | 20,6±2,1 | 17,1±2,6 | 9,4 ±1,6 | 5,6 ±1,6 | 1,0±2,6 | 1,7 ±2,4 |
| Δ $U_K$ m mol | Fu. | -1,5±0,34 | 1,3 ±0,74 | 0,4 ±0,73 | 1,2 ±0,62 | 1,4 ±0,92 | 2,4 ±0,67 | -1,3±0,52 | 0,01 ±0,30 |
| | Fu.ret. | -0,8±0,38 | -0,6 ±0,49 | 2,5 ±0,84 | 1,2 ±0,60 | -0,8 ±0,82 | -0,4 ±0,66 | -1,0±0,81 | -0,6 ±0,69 |
| | Fu.ret./Tri | -2,6±0,41 | -2,6 ±0,49 | -2,9 ±0,76 | 1,9 ±0,77 | -1,6 ±0,78 | -1,4 ±0,51 | -2,2±0,75 | 0,5 ± 0,92 |
| $U_{Na^+}/K^+$ | Fu. | 5,0±1,01 | 8,2± 1,14 | 6,2 ±1,22 | 5,1 ±0,80 | 5,6 ±0,67 | 4,1 ±0,62 | 6,3±0,72 | 6,3±0,97 |
| | Fu.ret. | 2,4±0,31 | 3,8± 0,59 | 3,3±0,29 | 4,3 ±0,48 | 5,3 ±0,60 | 5,0 ±0,43 | 6,2±0,60 | 6,8±1,10 |
| | Fu.ret./Tri | 6,2±0,74 | 14,5±2,52 | 11,6±1,71 | 14,3±2,94 | 13,1±2,01 | 9,6 ±1,75 | 10,1±0,69 | 7,9±2,35 |
| Δ $U_{Cl}$ m mol | Fu. | 8,6±2,27 | 36,5±11,56 | 23,9±7,64 | 7,2±5,37 | 1,7±3,98 | 0,43±2,47 | -7,4±2,07 | -5,2±2,10 |
| | Fu.ret. | – | – | – | – | – | – | – | – |
| | Fu.ret./Tri | 3,6±0,40 | 11,8±5,05 | 15,0±2,36 | 14,1±2,08 | 8,3±0,90 | 9,5±3,12 | -0,3±2,36 | -0,2±2,06 |
| Δ $U_{Mg}$ m mol | Fu. | 0,33 ±0,10 | 0,39 ±0,09 | 0,24 ±0,05 | 0,16 ±0,11 | 0,07 ±0,03 | | | |
| | Fu.ret. | 0,26 ±0,04 | 0,30 ±0,05 | 0,27 ±0,05 | 0,16 ±0,06 | 0,08 ±0,04 | | | |
| | Fu.ret./Tri | 0,11 ±0,05 | 0,24 ±0,06 | 0,21 ±0,07 | 0,11 ±0,02 | 0,02 ±0,03 | | | |
| Δ $U_{Ca}$ m mol | Fu. | 0,38 ±0,15 | 0,74 ±0,18 | 0,05 ±0,09 | 0,09 ±0,09 | 0,00 ±0,09 | -0,01 ±0,10 | -0,22 ±0,07 | -0,06 ±0,09 |
| | Fu.ret. | – | – | – | – | – | – | – | – |
| | Fu.ret./Tri | 0,30 ±0,07 | 0,37 ±0,09 | 0,44 ±0,07 | 0,22 ±0,07 | 0,17 ±0,08 | 0,34 ±0,06 | -0,04 ±0,07 | -0,20 ±0,08 |

Figur 1a zeigt den Plasmaspiegel nach Furosemid retard bei 6 gesunden Probanden (n) nach Einnahme von Furosemid retard (30 mg) + Triamteren (50 mg).

Figur 1b zeigt die pharmakodynamische Wirkung von Furosemid (30 mg) (O-O) bzw. der Kombination Furosemid retard (30 mg) + Triamteren (50 mg) (●-●) im Hinblick auf die Natrium-Mehrausscheidung (Differenz zum Kontrolltag).

Pharmakokinetik von Furosemid retard und Pharmakodynamik der Kombination zeigen einen annähernd gleichen zeitlichen Verlauf, d. h. verzögerter aber doch rasch erreichter max. Blutspiegel mit relativ schnellem Wirkungseintritt. Das Maximum für Pharmakokinetik und Pharmakodynamik liegt bei ca. 4 Stunden ; danach fallen beide Kurven schnell ab, die Wirkungsdauer erschöft sich um die 12. Stunde. Zu diesem Zeitpunkt liegen die Plasmaspiegel für Furosemid im Bereich der Nachweisgrenze.

Figur 2 zeigt im oberen Teil (A) die absolute Urinausscheidung und im unteren Teil (B) die Differenzen gegenüber dem Vortag innerhalb der beiden Behandlungsgruppen, graphisch dargestellt. Aus beiden Kurven ist ersichtlich, daß Furosemid initial sehr stark aber nur kurz, die Kombination Furosemid retard + Triamteren initial ebenfalls stark aber deutlich länger wirkt.

Figur 3 zeigt die Plasma-Renin-Aktivität nach oraler Gabe von Furosemid (30 mg) bzw. Furosemid retard (30 mg) + Triamteren (50 mg) bei je 6 gesunden Probanden. Angegeben sind die Mittelwerte der prozentualen Änderungen zum Ausgangswert.

$x = p \leq 0{,}05$ $xx = p \leq 0{,}01$ (x = signifikant ; xx = sehr signifikant ; p = propabilitas/Wahrscheinlichkeit)

Figur 4 zeigt die Aldosteron-Konzentration im Plasma nach oraler Gabe von 30 mg Furosemid bzw. der Kombination Furosemid retard (30 mg) + Triamteren (50 mg) bei je 6 gesunden Probanden. Angegeben ist die prozentuale Änderung der Mittelwerte zum Ausgangswert.

$x = p \leq 0{,}05$

**Patentanspruch** (für die Vertragsstaaten : BE, CH, DE, FR, GB, IT, LI, LU, NL, SE)

Kaliumneutrales Saluretikum mit antihypertensiver Wirkung auf Basis von Furosemid und Triamteren dadurch gekennzeichnet, daß Furosemid in Retardform vorliegt mit einer Wirkstofffreigabe von 5 bis 10 % nach 1 Stunde bis > 85 % nach 8 h und daß die Wirkstoffe Furosemid in einer Menge von 10 bis 50 mg und Triamteren in einer Menge von 20 bis 60 mg vorliegen.

**Patentanspruch** (für den Vertragsstaat AT)

Verfahren zur Herstellung eines kaliumneutralen Saluretikums mit antihypertensiver Wirkung auf Basis von Furosemid und Triamteren, dadurch gekennzeichnet, daß man Furosemid in Retardform mit einer Wirkstofffreigabe von 5 bis 10 % nach 1 Stunde bis > 85 % nach 8 h und mit einer Wirkstoffmenge von 10 bis 50 mg mit Triamteren in einer Menge von 20 bis 60 mg kombiniert.

**Claim** (for the Contracting States : BE, CH, DE, FR, GB, IT, LI, LU, NL, SE)

Potassium neutral saluretic with antihypertensive activity based on furosemide and triamterene characterized in that the furosemide is present in a sustained-release form which releases 5 to 10 % of the active ingredient after 1 hour and up to > 85 % after 8 hours, and in that 10 to 50 mg of the active ingredient furosemide and 20 to 60 mg of the active ingredient triamterene are present.

**Claim** (for the Contracting State AT)

Process for the production of a potassium neutral saluretic with antihypertensive activity based on furosemide and triamterene, characterized in that furosemide, in a sustained-release form, which releases 5 to 10 % of the active ingredient after 1 hour and up to > 85 % after 8 hours and which contains 10 to 50 mg of the active ingredient is combined with 20 to 60 mg of triamterene.

**Revendication** (pour les Etats contractants : BE, CH, DE, FR, GB, IT, LI, LU, NL, SE)

Salurétique, neutre vis-à-vis du potassium, à activité antihypertensive, à base de Furosemid et de Triamteren, caractérisé en ce que le Furosemid est présent sous une forme retard avec une libération de substance active allant de 5 à 10 % après 1 heure jusqu'à plus de 85 % après 8 heures, et en ce que les substances actives sont présentes en des quantités allant de 10 à 50 mg pour le Furosemid et de 20 à 60 mg pour le Triamteren.

**Revendication** (pour l'Etat contractant AT)

Procédé de préparation d'un salurétique neutre vis-à-vis du potassium et à activité antihypertensive, à base de Furosemid et de Triamteren, caractérisé en ce que l'on combine, avec une quantité allant de 20 à 60 mg de Triamteren, du Furosemid sous forme retard avec une libération de substance active allant de 5 à 10 % après 1 heure jusqu'à plus de 85 % après 8 heures, en une quantité de substance active allant de 10 à 50 mg.

FIG.1a

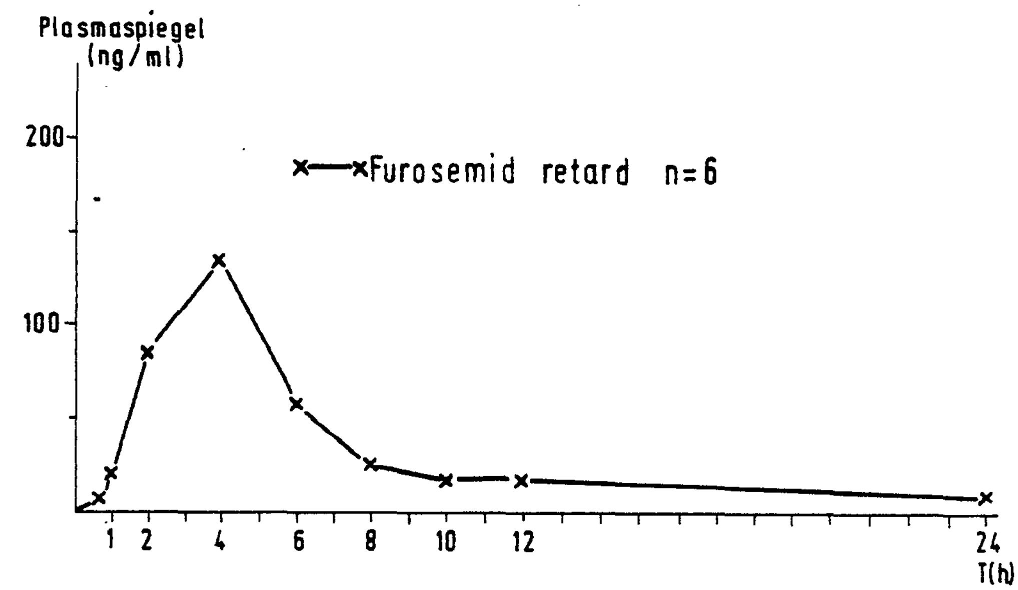
Plasmaspiegel (ng/ml)
200
100
Furosemid retard n=6
1
2
4
6
8
10
12
24
T(h)

FIG.1b

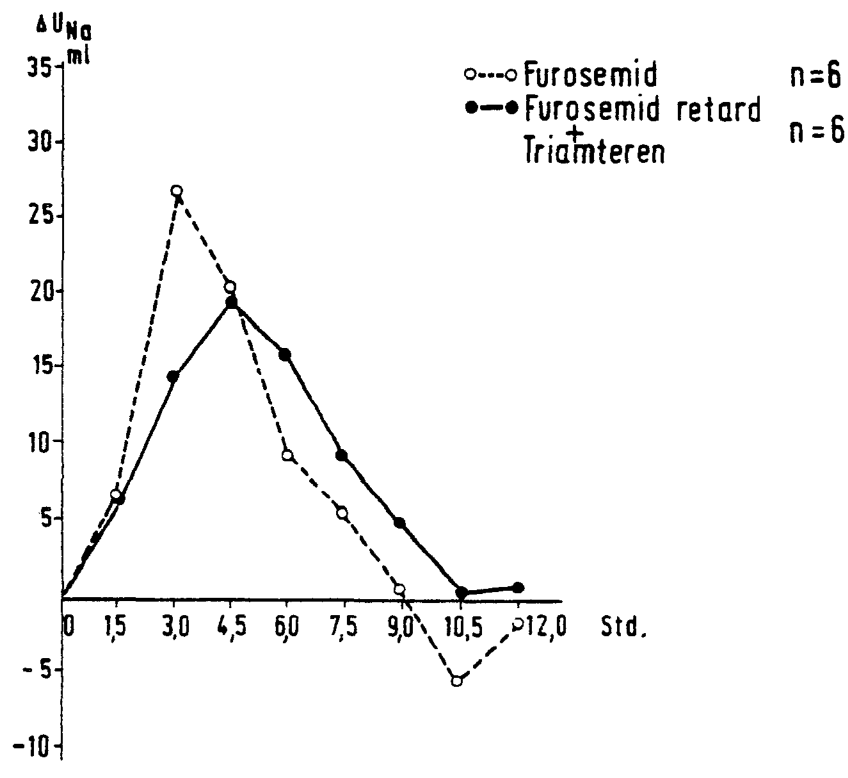
ΔU Na ml
35
30
25
20
15
10
5
-5
-10
Furosemid n=6
Furosemid retard + Triamteren n=6
0
1,5
3,0
4,5
6,0
7,5
9,0
10,5
12,0
Std.

FIG. 2

(A)

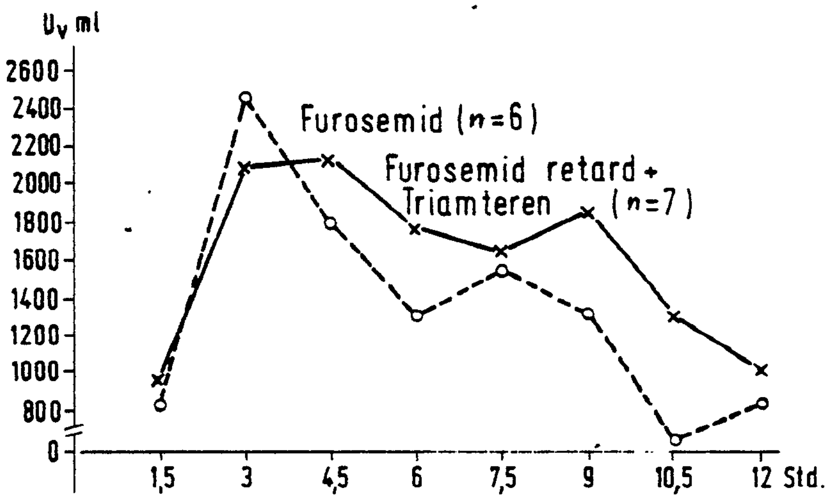
Uv ml
2600
2400
2200
2000
1800
1600
1400
1200
1000
800
0
1,5
3
4,5
6
7,5
9
10,5
12 Std.
Furosemid (n=6)
Furosemid retard +
Triamteren (n=7)

(B)

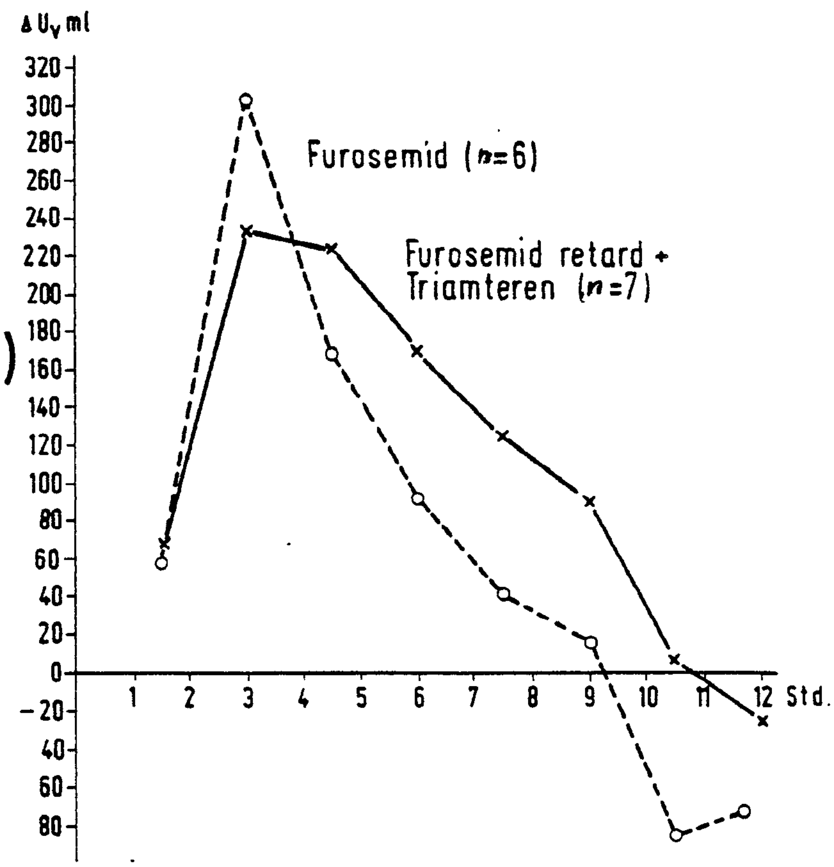
Δ Uv ml
320
300
280
260
240
220
200
180
160
140
120
100
80
60
40
20
0
-20
40
60
80
1 2 3 4 5 6 7 8 9 10 11 12 Std.
Furosemid (n=6)
Furosemid retard +
Triamteren (n=7)

FIG.3

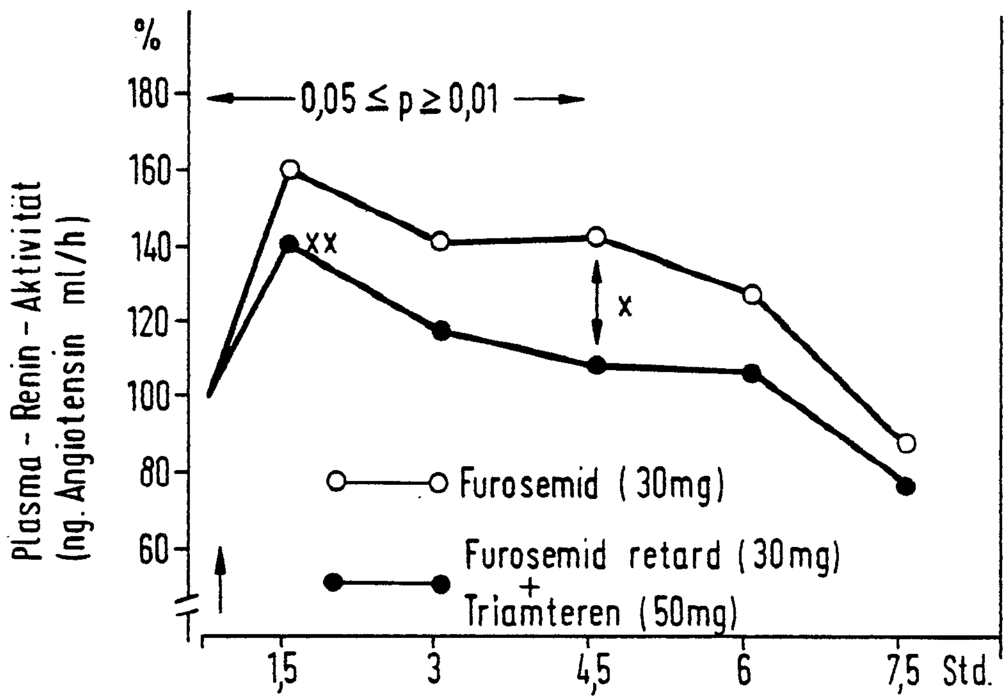
%
180
160
140
120
100
80
60
0,05 ≤ p ≥ 0,01
xx
x
Plasma - Renin - Aktivität
(ng. Angiotensin ml/h)
Furosemid (30mg)
Furosemid retard (30mg)
+
Triamteren (50mg)
1,5
3
4,5
6
7,5 Std.

FIG. 4

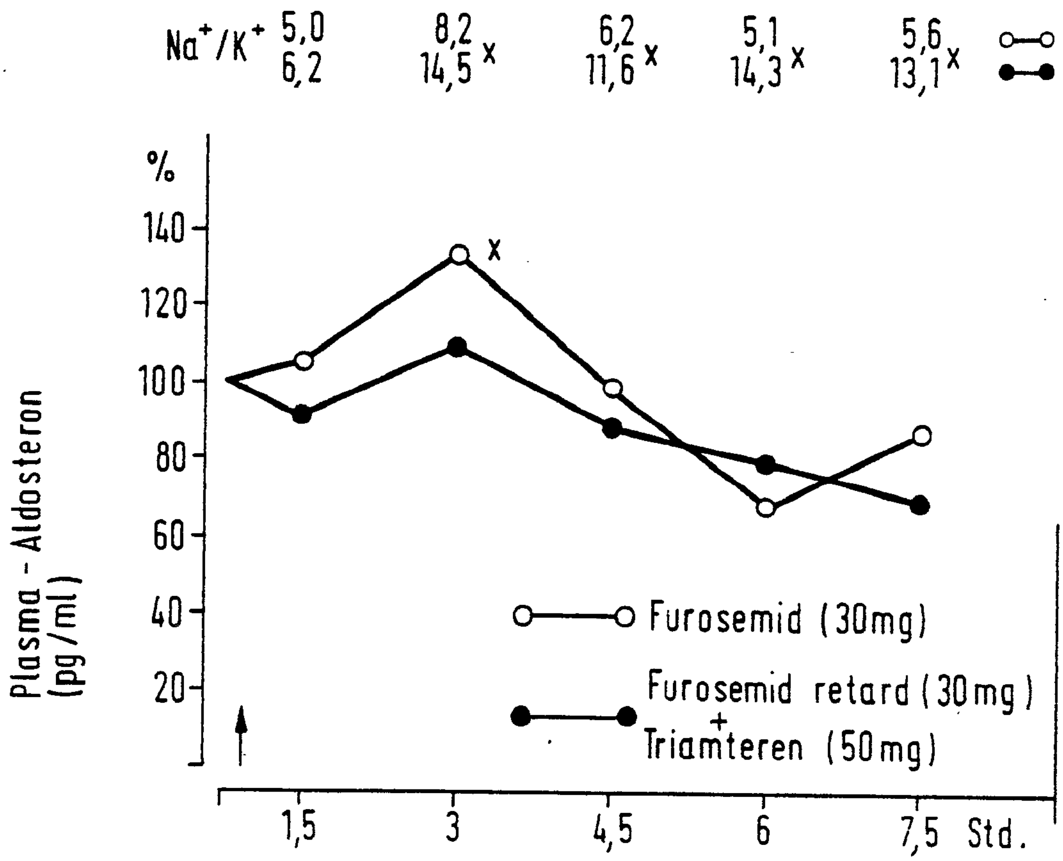

Na⁺/K⁺ 5,0 6,2
8,2 14,5 x
6,2 11,6 x
5,1 14,3 x
5,6 13,1 x
%
140
120
100
80
60
40
20
Plasma - Aldosteron (pg/ml)
x
Furosemid (30mg)
Furosemid retard (30mg) + Triamteren (50mg)
1,5
3
4,5
6
7,5 Std.